# FASCICULE DE BREVET EUROPEEN

(11) **EP 3 446 116 B1**
(45) Date de publication et mention de la délivrance du brevet: **09.08.2023**
(21) Numéro de dépôt: 16724440.9
(22) Date de dépôt: 20.04.2016
(51) Int. Cl.: G01N 33/28, G01N 21/64

(54) **MÉTHODE DE DOSAGE D'ADDITIFS UTILISÉS POUR LA RÉCUPÉRATION ASSISTÉE DU PÉTROLE ET DU GAZ DE SCHISTE**
VERFAHREN ZUM NACHWEIS UND ZUR QUANTIFIZIERUNG VON VERWENDETEN ADDITIVEN IN DER TERTIÄREN GEWINNUNG VON ÖL UND SCHIEFERGAS
METHOD FOR DETECTING AND QUANTIFYING ADDITIVES USED IN THE ENHANCED RECOVERY OF OIL AND SHALE GAS

(43) Date de publication de la demande: 27.02.2019
(73) Titulaire: TotalEnergies OneTech, 92400 Courbevoie (FR); Université Claude Bernard Lyon 1, 69622 Villeurbanne (FR); Centre National de la Recherche Scientifique CNRS, 75016 Paris (FR)
(72) Inventeur: MARTINI, Matteo, 01330 Villars Les Dombes (FR); TILLEMENT, Olivier, 69270 Fontaines Saint Martin (FR); MARAIS, Arthur, 56650 Lochrist (FR); HURTEVENT, Christian, 81000 Albi (FR); JOUENNE, Stéphane, 64320 Bizanos (FR)
(74) Mandataire: Plasseraud IP
(86) Numéro de dépôt international: PCT/FR2016/050912
(87) Numéro de publication internationale: WO 2017/182720

(56) Documents cités:
- WO-A1-2011/055038
- WO-A1-2015/075299
- Thomas Brichart: "Traceurs fluorescents à base de lanthanides en milieu complexe", , 7 juillet 2014 (2014-07-07), XP055284289, Extrait de l'Internet: URL:https://tel.archives-ouvertes.fr/tel-0 1066086/document [extrait le 2016-06-28]
- Thomas Brichart ET AL: "IPTC-17933-MS The Use of Fluorescent Tracers for Inhibitor Concentration Monitoring Useful for Scale Inhibitor Squeeze Evaluation", , 10 décembre 2014 (2014-12-10), XP055257208, Extrait de l'Internet: URL:https://www.onepetro.org/conference-pa per/IPTC-17933-MS [extrait le 2016-03-10]

## Description

### DOMAINE TECHNIQUE

La présente invention a pour objet une méthode de détection et quantification d'additifs utilisés pour la récupération assistée du pétrole et du gaz de schiste, dans un fluide aqueux complexe. En particulier, la présente invention a pour objet une méthode de détection et quantification de polymères hydrosolubles utilisés pour la récupération assistée du pétrole et du gaz de schiste, dans un fluide aqueux complexe.

### ARRIERE-PLAN DE L'INVENTION

Il est bien connu dans l'exploitation pétrolière d'un gisement, que le plus souvent, on n'extrait pas plus de la moitié, voire moins, du pétrole présent à l'origine dans le gisement et qu'il est nécessaire de procéder à plusieurs méthodes de récupération avancées pour maximiser la proportion de pétrole extraite.

La récupération par les moyens primaires, c'est-à-dire l'utilisation de l'énergie d'extraction résultant de gaz ou de liquides présents dans le sous-sol sous l'effet d'une certaine pression dans le gisement, permet seulement d'extraire de faibles pourcentages du pétrole total présent dans le gisement.

Après cette récupération primaire, on procède à une autre récupération, la récupération assistée du pétrole. Différents puits sont forés dans le réservoir, des puits d'injection et des puits de production. Dans la plupart des cas, de l'eau est injectée dans le réservoir via les puits d'injection. Ceci a pour effet de maintenir la différence de pression avec la surface et ainsi d'assurer le flux du pétrole dans le réservoir, le pétrole étant poussé et remplacé par l'eau injectée. Cependant, il est fréquent que la mobilité de l'eau soit supérieure à celle du pétrole, ce qui engendre des fronts instables entre les deux liquides non miscibles et des phénomènes de digitation visqueuse. En conséquence, le rendement de balayage à l'échelle macroscopique reste faible, une grande partie du gisement n'est pas balayée par l'eau injectée et une quantité importante de pétrole reste dans le réservoir. Augmenter la viscosité du liquide injecté permet de diminuer le rapport de mobilité entre le liquide injecté et le pétrole et d'augmenter le rendement de balayage à l'échelle macroscopique. Ainsi, des additifs sont ajoutés à l'eau injectée afin d'accroître sa viscosité. En rendant l'eau moins mobile, un meilleur contrôle de mobilité entre le liquide injecté et le pétrole peut être obtenu.

Parmi les additifs utilisés, on peut citer les polymères hydrosolubles permettant d'accroître la viscosité de l'eau, comme les polyacrylamides ou le xanthane. Ces polymères ont une masse moléculaire élevée, typiquement comprise entre 1 et 30 MDa, afin d'accroître la viscosité de l'eau à moindre coût. Les polymères utilisés dans la récupération assistée du pétrole et du gaz de schiste sont donc distincts des polymères utilisés comme inhibiteur de dépôt ou de corrosion, qui ont des masses moléculaires plus faibles. Ils sont généralement utilisés sous forme de solutions aqueuses et sont notamment décrits dans la revue « Polymers for enhanced oil recovery : A paradigm for structure-property relationship in aqueous solution », Progress in Polymer Science, Vol 36, pp. 1558-1628, 2011.

Cependant, les conditions dans les puits de forage et les réservoirs sont telles qu'elles peuvent entraîner la dégradation des additifs utilisés dans la récupération assistée du pétrole et du gaz de schiste, ce qui résulte en une perte d'efficacité. La dégradation des additifs peut être estimée par la mesure de la viscosité de la solution et de la concentration en additifs. Ainsi, il est intéressant de pouvoir doser (détection et/ou quantification) ces additifs au niveau des puits d'injection et de production afin de déterminer leur niveau de dégradation. Cela permet d'estimer la dégradation des additifs et, en cas de besoin, d'injecter une quantité supplémentaire d'additifs, ajustée pour tenir compte des contraintes économiques du procédé et de son impact environnement et d'optimiser la récupération assistée du pétrole et du gaz de schiste.

Les méthodes utilisées actuellement pour doser les additifs utilisés dans la récupération assistée du pétrole et du gaz de schiste sont souvent peu précises et/ou longues et nécessitent des appareillages souvent insuffisamment adaptés aux conditions d'exploitation.

La mise au point d'une méthode de détection fiable et rapide se heurte en outre à la présence dans le fluide constitué des eaux d'exploitation d'une diversité de composés tels que des sels et des résidus organiques. Du fait de la présence de ces composés, le fluide complexe produit présente notamment une fluorescence intrinsèque qui empêche la détection de ces additifs, éventuellement marqués par une sonde fluorescente, à l'aide des techniques traditionnelles de fluorescence. Enfin, les sites de production sont généralement situés dans des lieux reculés, éloignés des laboratoires d'analyse locaux, ce qui constitue une contrainte supplémentaire.

Il serait donc souhaitable de pouvoir doser les additifs utilisés dans la récupération assistée du pétrole et du gaz de schiste directement sur site, dans les eaux d'injection ou de production, à l'aide d'une méthode simple, fiable et précise, utilisable sur une diversité d'additifs et pouvant être mise en oeuvre à l'aide d'appareils peu encombrants pour pouvoir être déplacés aisément.

Les inventeurs ont démontré que ces besoins pouvaient être satisfaits en mélangeant les fluides à analyser, susceptibles de contenir des additifs utilisés dans la récupération assistée du pétrole et du gaz de schiste, à une solution révélatrice comprenant au moins un ion lanthanide et éventuellement au moins un agent chélateur de l'ion lanthanide, et en utilisant la méthode de fluorescence en temps résolu. Cette méthode permet en effet de s'affranchir de la fluorescence naturelle des eaux d'exploitation, qui présente des temps très courts d'émission, et de ne récolter que la lumière émise après un délai de quelques microsecondes à une milliseconde, de préférence de 100 microsecondes à une milliseconde, résultant de la fluorescence des additifs utilisés dans la récupération assistée du pétrole ainsi marqués. En outre, la possible pré-complexation des ions lanthanides avec un agent chélateur permet d'améliorer la sensibilité de détection des additifs utilisés dans la récupération assistée du pétrole, et ce, même malgré l'augmentation de la fluorescence des ions lanthanides liée à la présence de l'agent chélateur. La présente méthode permet également de détecter des additifs utilisés dans la récupération assistée du pétrole à faible pouvoir complexant ou excitable facilement dans le proche UV/visible.

Cette méthode a en outre pour avantage d'identifier spécifiquement le type d'additif utilisé dans la récupération assistée du pétrole, sans étape de marquage préalable et même lorsqu'il est présent dans un fluide complexe formé par les eaux de production dans le milieu pétrole ou gaz de schiste, en fonction de sa signature optique, en exploitant simultanément les spectres d'excitation et d'émission et les durées de vie des signaux émis.

Dans sa thèse de doctorat "Traceurs fluorescents à base de lanthanides en milieu complexe", 7 juillet 2014 (2014-07-07), XP055284289, Thomas Brichart décrit aussi une méthode de détection d'additifs utilisés dans la récupération assistée.

### RESUME DE L'INVENTION

La présente invention a pour objet une méthode de détection d'additifs utilisés pour la récupération assistée du pétrole et du gaz de schiste, dans des eaux d'injection ou des eaux de production, ladite méthode comprenant:
a. le mélange d'une solution révélatrice comprenant au moins un cation lanthanide et éventuellement un agent chélateur des lanthanides, avec un échantillon d'eaux d'injection ou d'eaux de production à analyser comprenant au moins un additif utilisé pour la récupération assistée du pétrole et du gaz de schiste, dans des conditions permettant la complexation du lanthanide par l'additif présent,
b. La détection et, le cas échéant, la quantification, de la variation de fluorescence liée à la présence de l'additif dans les eaux d'injection ou les eaux de production par fluorescence en temps résolu,
ledit additif utilisé pour la récupération assistée du pétrole et du gaz de schiste étant un polymère hydrosoluble ayant une masse molaire comprise entre 1 MDa et 30 MDa choisi parmi :
- les polymères comprenant au moins un motif de répétition comprenant une liaison amide ;
- les biopolymères anioniques tels que le xanthane ;
- les polymères cationiques, tel que le poly DADMAC.

### DESCRIPTION DETAILLEE DE MODES DE REALISATIONS

### Définitions

Par « un groupe Cₓ à C_{y} alkyl », on entend, au sens de la présente invention et dans le texte qui suit, une chaîne alkyle linéaire ou ramifiée, ou un cycloalkyl, ayant x à y atomes de carbone.

Par « un groupe C₁ à C₄ alkyl », on entend, au sens de la présente invention et dans le texte qui suit, une chaîne alkyle linéaire ou ramifiée, ou un cycloalkyl, ayant 1 à 4 atomes de carbone. Par exemple on peut citer les chaînes alkyles linéaires suivantes : méthyl, éthyl, n-propyl et n-butyl. Comme exemple de chaîne alkyle ramifiée, on peut citer les groupes suivants : *iso-*propyl, isobutyl, sec-butyl et tert-butyl.

Par « un groupe C₁ à C₁₀ alkyl », on entend, au sens de la présente invention et dans le texte qui suit, une chaîne alkyle linéaire ou ramifiée, ou un cycloalkyl, ayant 1 à 10 atomes de carbone. Par exemple on peut citer les chaînes alkyles linéaires suivantes : méthyl, éthyl, n-propyl, n-butyl, n-pentyle, n-hexyl, n-heptyl, n-octyl, n-nonyl et n-décyle. Comme exemple de chaîne alkyle ramifiée, on peut citer les groupes ci-dessus et les groupes suivants : isopentyl, 2,2-diméthylpropyl, *iso-*octyl, iso-nonyl et iso-décyle.

Par « substitué ou non », on entend, au sens de la présente invention et dans le texte qui suit, que le groupe alkyl ou aryl peut être substitué par un ou plusieurs groupes fonctionnels, par exemple choisi parmi les groupes amine, imine, nitro, cyano, amide, imide, hydroxyle, alcoxyle, carbonyle, carboxyle, ester, thiol, thioéther, thioester et halogénure.

### Solution révélatrice pour la mise en œuvre de la méthode de détection d'additifs

La mise en oeuvre de la méthode de détection comprend l'utilisation d'une solution révélatrice comprenant au moins un cation lanthanide et éventuellement au moins un agent chélateur des lanthanides.

Le cation lanthanide utilisable dans la solution révélatrice peut être choisi parmi les éléments de numéro atomique 57 (lanthane) à 71 (lutétium), tels que Pr, Nd, Sm, Eu, Tb, Dy, Ho, Er, Tm et Yb, ainsi que leurs mélanges. La présente méthode est particulièrement adaptée pour une solution révélatrice comprenant l'Europium. Avantageusement, la mesure de la fluorescence émise par l'Europium peut être effectuée au travers de cellules plastiques jetables, sans la nécessité d'utiliser des cellules transparentes dans l'UV (comme les cellules en quartz ou silice).

Dans un mode de réalisation spécifique, la solution révélatrice comprend en outre au moins 1g/L d'ions chlorures, et de préférence une concentration en ions chlorures comprise entre 5 et 50g/L.

Dans un autre mode de réalisation particulier pouvant être combiné avec les précédents, la solution révélatrice comprend en outre au moins 1g/L d'un composé à fort pouvoir tampon, utilisé dans la fabrication de solution tampon, et permettant de maintenir la valeur du pH après ajout de 1/10 d'eau à analyser dans un intervalle de pH inférieur à 0,5 unité, par exemple l'acide 4-(2-hydroxyéthyl)-1-pipérazine éthane sulfonique (HEPES) ou encore l'acétate de sodium.

La solution révélatrice comprend éventuellement un agent chélateur des lanthanides. Par « agent chélateur des lanthanides », on entend un ion ou une molécule portant des fonctions chimiques lui permettant de se lier à un ou plusieurs atomes ou ions centraux dudit lanthanide et dont l'interaction lanthanide/ligand ainsi formée est supérieure à l'interaction lanthanide/eau et de préférence supérieure à l'interaction lanthanide/chlorure, permettant ainsi une pré-complexation des ions lanthanides en diminuant le nombre de molécules d'eau présentes dans la sphère de coordination du lanthanide, il en résulte une diminution de l'effet quenching de l'eau sur la fluorescence de l'ion lanthanide (DeW. Horrocks et al., JACS 1979 101 :2, 334-340) .

De préférence les agents chélateurs des lanthanides utilisables dans la solution révélatrice sont choisis parmi :
(i) les molécules comprenant au moins une fonction amine, de préférence deux fonctions amine, et/ou
(ii) les molécules comprenant au moins une fonction acide carboxylique, en particulier l'acide maléique ou les dérivés polymériques d'acide maléique.

Dans un mode de réalisation spécifique, l'agent chélateur comprend en outre au moins un groupement aryle, de préférence un groupement hétérocyclique simple, substitué ou non, par exemple un groupement pyridine et ses dérivés substitués. Outre l'effet chélateur apporté par la fonction amine ou acide carboxylique, la présence d'un groupement aryle et par exemple un groupement pyridine permet en plus d'amplifier le signal de fluorescence par effet antenne (Armelao, L.; et al. COORDINATION CHEMISTRY REVIEWS Volume: 254 Issues: 5-6 Spécial Issue: SI Pages: 487-505 Published: MAR 2010).

Dans un mode de réalisation préféré, l'agent chélateur est choisi parmi la diaminopyridine, l'imidazoline, le polyanhydride maléique hydrolysé, les polyacides carboxyliques, l'EDTA, l'acide oxalique, l'acétylacétonate, le thiodiacétate, l'acide nitrilotriacétique (NTA), leurs dérivés ou leurs mélanges.

Le rapport de concentration massique dans la solution révélatrice entre l'agent chélateur et le lanthanide est compris entre 1 :10 et 10 :1, par exemple entre 1 :3 et 3 :1.

L'invention a trait en particulier à une solution révélatrice pour la détection d'additifs utilisés pour la récupération assistée du pétrole et du gaz de schiste, comprenant :
i. un cation lanthanide, par exemple Eu3+,
ii. des ions chlorure à plus de 1 g/L ;
iii. le cas échéant, un agent chélateur des lanthanides, par exemple choisi parmi la diaminopyridine, l'imidazoline, le polyanhydride maléique hydolysé, les polyacides carboxyliques, l'acide oxalique, l'acétylacétonate, le thiodiacétate, ou leurs dérivés, l'EDTA, l'acide nitrilotriacétique (NTA) ;
iv. le cas échéant, un composé chimique utilisé classiquement dans la fabrication des solutions tampon par exemple l'acide 4-(2-hydroxyéthyl)-1-pipérazine éthane sulfonique (HEPES) à plus de 1g/L;
le cas échéant, le rapport de concentration entre l'agent chélateur et le lanthanide étant compris entre 1 :10 et 10 :1, de préférence entre 1 :3 et 3 :1.

Dans un mode de réalisation particulier, la solution révélatrice comprend entre 1 et 10.000ppm de cations lanthanide, en particulier issus de EuCl₃.6H₂O, 10 et 5000ppm de 2,5-diaminopyridine et entre 1 et 50g/L de NaCl, à pH compris entre 4 et 8.

La présente invention vise les solutions révélatrices telles que définies ci-dessus pour leurs utilisations dans une méthode de détection d'additifs utilisés pour la récupération assistée du pétrole et du gaz de schiste comme définie ci-dessous.

### Fluide aqueux à analyser, extraction et mélange avec la solution révélatrice

La méthode de détection d'additifs utilisés pour la récupération assistée du pétrole et du gaz de schiste comprend l'extraction d'un échantillon (fluide aqueux) à analyser et son mélange avec la solution révélatrice définie ci-dessus.

La méthode vise la détection d'additifs utilisés pour la récupération assistée du pétrole et du gaz de schiste, en particulier dans un extrait d'un fluide pétrolier contenant de l'eau, par exemple, un extrait d'un puits de production de pétrole ou de gaz de schiste. Dans un mode préféré de la méthode selon l'invention, on extrait un échantillon de fluide aqueux à analyser d'un puits de production de pétrole ou de gaz de schiste ou d'une eau industrielle prélevée au cours du processus d'exploitation et/ou de fabrication.

De préférence, l'additif utilisé pour la récupération assistée du pétrole et du gaz de schiste est contenu dans le fluide aqueux à une concentration inférieure ou égale à 500 ppm, de préférence inférieure ou égale à 10 ppm, encore plus préférentiellement inférieure ou égale à 1 ppm, par exemple comprise entre 100 ppb et 500 ppm, ou entre 100 ppb et 10 ppm ou encore entre 10 ppb et 1 ppm.

Dans la méthode selon l'invention, le volume extrait de l'échantillon à analyser (par exemple extrait de puits de pétrole ou de gaz de schiste) peut être par exemple compris entre 0,1 mL et 1 litre, 5 litres, 10 litres ou plus.

Le volume extrait peut être traité avant analyse, par exemple par acide/base/oxydation/précipitation ou par des étapes de filtration ou sédimentation afin d'éliminer certains composés indésirables, avant le mélange avec la solution révélatrice.

On mélange cet échantillon (par exemple extrait de puits de pétrole ou de gaz de schiste) avec la solution révélatrice, par exemple dans des proportions de 1 :100 à 100 :1 vol/vol entre le volume de l'échantillon à analyser et le volume de solution révélatrice et de préférence compris entre 1 :20 et 1 :5, par exemple autour de 1 :10.

Dans un mode de réalisation particulier, les ions lanthanides pré-complexés avec les agents chélateurs présents dans la solution révélatrice forment des complexes avec les additifs utilisés pour la récupération assistée du pétrole et du gaz de schiste éventuellement présents dans l'échantillon. Il en résulte une diminution de l'effet quenching des molécules d'eau autour des cations lanthanides et donc une augmentation de la fluorescence des cations lanthanides.

### Additifs utilisés dans la récupération assistée du pétrole et du gaz de schiste

Dans un mode de réalisation, l'additif utilisé pour la récupération assistée du pétrole et du gaz de schiste ne présente pas de fluorescence intrinsèque. Avantageusement, avec la méthode selon la présente invention, l'additif à détecter n'a pas été couplé à un agent chélateur avant son injection ou un agent luminescent ou autre agent de marquage.

Les additifs utilisés pour la récupération assistée du pétrole et du gaz de schiste susceptibles d'être présents dans l'échantillon à analyser, et détectables par la méthode de détection selon l'invention, sont notamment des polymères, de préférence des polymères hydrosolubles. Par « polymères », on entend les polymères synthétiques et les biopolymères (polymères issus de la biomasse).

Les polymères utilisés pour la récupération assistée du pétrole et du gaz de schiste peuvent être des polymères linéaires, en étoile ou en peigne.

Il peut s'agir de polymères comprenant un seul motif de répétition (homopolymères) ou plusieurs motifs de répétition (copolymères), par exemple deux motifs de répétition (bipolymères), trois motifs de répétition (terpolymères), quatre motifs de répétition ou plus.

Les polymères comprenant plusieurs motifs de répétition peuvent être des copolymères à blocs, des copolymères statistiques ou des copolymères alternés. Il peut également s'agir de copolymères greffés.

Avantageusement, le polymère utilisé pour la récupération assistée du pétrole et du gaz de schiste est un copolymère statistique linéaire.

Dans un mode de réalisation de l'invention, le polymère est un polymère non ionique, anionique ou zwitterionique, de préférence anionique.

De manière préférée, le polymère utilisé pour la récupération assistée du pétrole et du gaz de schiste est un polymère comprenant au moins un motif de répétition comprenant une liaison amide.

Selon l'invention, l' additif utilisé pour la récupération assistée du pétrole et du gaz de schiste est choisi parmi :
- les polymères comprenant au moins un motif de répétition comprenant une liaison amide ;
- les biopolymères anioniques tels que le xanthane ;
- les polymères cationiques, tel que les polymères à base de chlorure de diallyldiméthylammonium (les polyDADMAC).

Avantageusement, le polymère comprenant au moins un motif de répétition comprenant une liaison amide comprend un motif de répétition de formule I 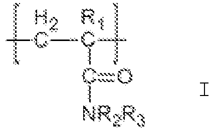
où R₁ est -H ou -CH₃,
R₂ est -H ou un groupe C₁ à C₄ alkyl substitué ou non,
R₃ est -H ou un groupe C₁ à C₄ alkyl substitué ou non, ou un groupe -L-R₄,
où L est une liaison ou un groupe C₁ à C₁₀ alkyl substitué ou non, interrompu par 0, 1 ou plusieurs liaisons -NR₂- ou -O- ou -S-, ou un groupe - (C₁ à C₁₀ alkyl substitué ou non) - (N⁺R₆R₇) - (C₁ à C₁₀ alkyl substitué ou non) - avec R₆ et R₇ qui sont indifféremment -H ou un groupe C₁ à C₄ alkyl substitué ou non,
et R₄ est -H ou un groupe carboxylate (-COO⁻) ou un groupe sulfonate (-SO₃⁻), avec éventuellement un contre-ion.

De manière préférée, le polymère comprenant au moins un motif de répétition comprenant une liaison amide comprend un motif de répétition de formule I
où R₁ est -H ou -CH₃,
R₂ est -H ou un groupe C₁ à C₄ alkyl substitué ou non,
R₃ est -H ou un groupe C₁ à C₄ alkyl substitué ou non, ou un groupe -L-R₄,
où L est une liaison ou un groupe C₁ à C₁₀ alkyl substitué ou non, interrompu par 0, 1 ou plusieurs liaisons -NR₂- ou -O- ou -S-,
et R₄ est -H ou un groupe carboxylate (-COO⁻) ou un groupe sulfonate (-SO₃⁻), avec éventuellement un contre-ion.

De manière particulièrement préférée, le polymère comprenant au moins un motif de répétition comprenant une liaison amide comprend un motif de répétition de formule I
où R₁ est -H,
R₂ est -H,
R₃ est -H ou un groupe -L-R₄,
où L est une liaison ou un groupe C₁ à C₁₀ alkyl substitué ou non, interrompu par 0, 1 ou plusieurs liaisons -NR₂- ou -O- ou -S-,
et R₄ est un groupe sulfonate (-SO₃⁻) avec un contre-ion.

Lorsque le groupe R₄ comprend un contre-ion, celui-ci est de préférence choisi parmi H⁺, les cations de métaux alcalins et les ammoniums primaires, secondaires et tertiaires. Le contre-ion peut également être choisi parmi Na⁺, K⁺, Li⁺, NH₄⁺, Zn⁺, Ca²⁺, Zn²⁺, Al³⁺ et Mg²⁺.

Par exemple, le polymère comprend un motif de répétition de formule I où R₁, R₂ et R₃ sont -H ou un motif de répétition de formule I où R₁ et R₂ sont -H et R₃ est -L-R₄ où L est un groupe tert-butyl et R₄ est groupe sulfonate (-SO₃⁻), avec un contre-ion, de préférence Na+.

Dans un mode de réalisation de l'invention, le polymère comprenant au moins un motif de répétition de formule I comprend en outre un motif de répétition de formule II 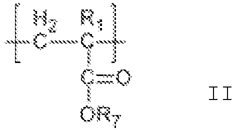
où R₁ est -H ou -CH₃,
et OR₇ est O-H ou O⁻ et un contre-ion, ledit contre-ion étant de préférence choisi parmi H⁺, les cations de métaux alcalins et les ammoniums primaires, secondaires et tertiaires. Le contre-ion peut également être choisi parmi Na⁺, K⁺, Li⁺, NH₄⁺, Zn⁺, Ca²⁺, Zn²⁺, Al³⁺ et Mg²⁺.

Par exemple, le polymère est un copolymère comprenant un motif de répétition de formule I où R₁, R₂ et R₃ sont -H et un motif de répétition de formule II où R₁ est -H et OR₇ est O⁻Na⁺. Ce copolymère comprend au minimum 25 mol% de motif de répétition de formule II, par exemple entre 25 et 75 mol%.

Dans un autre mode de réalisation particulier pouvant être combiné avec les précédents, le polymère comprenant un motif de répétition de formule I comprend en outre un motif de répétition de formule III 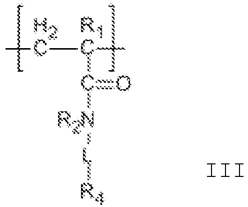
où R₁ est -H ou -CH₃,
R₂ est -H ou un groupe C₁ à C₄ alkyl substitué ou non,
L est une liaison ou un groupe C₁ à C₁₀ alkyl substitué ou non, interrompu par 0, 1 ou plusieurs liaisons -NR₂- ou -O- ou -S-, ou un groupe - (C₁ à C₁₀ alkyl substitué ou non) - (N⁺R₆R₇) - (C₁ à C₁₀ alkyl substitué ou non) - avec R₆ et R₇ qui sont indifféremment -H ou un groupe C₁ à C₄ alkyl substitué ou non,
et R₄ est -H ou un groupe carboxylate (-COO⁻) ou un groupe sulfonate (-SO₃⁻), avec éventuellement un contre-ion.

De manière préférée, le polymère comprenant un motif de répétition de formule I comprend en outre un motif de répétition de formule III
où R₁ est -H ou -CH₃,
R₂ est -H ou un groupe C₁ à C₄ alkyl substitué ou non,
R₃ est -H ou un groupe C₁ à C₄ alkyl substitué ou non, ou un groupe -L-R₄,
où L est une liaison ou un groupe C₁ à C₁₀ alkyl substitué ou non, interrompu par 0, 1 ou plusieurs liaisons -NR₂- ou -O- ou -S-,
et R₄ est -H ou un groupe carboxylate (-COO⁻) ou un groupe sulfonate (-SO₃⁻), avec éventuellement un contre-ion.

De manière particulièrement préférée, le polymère comprenant un motif de répétition de formule I comprend en outre un motif de répétition de formule III
où R₁ est -H,
R₂ est -H,
R₃ est -H ou un groupe -L-R₄,
où L est une liaison ou un groupe C₁ à C₁₀ alkyl substitué ou non, interrompu par 0, 1 ou plusieurs liaisons -NR₂- ou -O- ou -S-,
et R₄ est un groupe sulfonate (-SO₃⁻) avec un contre-ion.

Par exemple, le polymère est un copolymère comprenant un motif de répétition de formule I où R₁, R₂ et R₃ sont -H et un motif de répétition de formule III où R₁ et R₂ sont -H, L est un groupe *tert*-butyl et R₄ est groupe sulfonate (-SO₃⁻), avec un contre-ion, de préférence Na⁺.

Dans un autre mode de réalisation particulier pouvant être combiné avec les précédents, le polymère comprenant un motif de répétition de formule I comprend en outre un motif de répétition issu de la polymérisation d'un monomère non-ionique. De préférence ledit monomère non-ionique est choisi parmi acryloyl morpholine, N-vinylcaprolactame, N-vinylpyrrolidone, N,N-diméthylacrylamide, N-isopropylacrylamide, diacétone acrylamide, N-vinylformamide, N-vinyl acetamide, N-vinylpyridine, hydroxybutyl vinyl ether et isoprenol. De façon particulièrement préférée, le monomère non-ionique est N-vinylpyrrolidone.

Par exemple, le polymère est un copolymère comprenant un motif de répétition de formule I où R₁, R₂ et R₃ sont -H, un motif de répétition de formule III où R₁ et R₂ sont - H, L est un groupe *tert*-butyl et R₄ est groupe sulfonate (-SO₃⁻), avec un contre-ion, de préférence Na⁺ et un motif répétition issu de la polymérisation de N-vinylpyrrolidone.

Dans un autre mode de réalisation particulier pouvant être combiné avec les précédents, le polymère comprenant un motif de répétition de formule I comprend en outre un motif de répétition comprenant un groupement hydrophobe, c'est-à-dire non-polaire, de préférence de formule IV 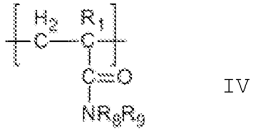
où R₁ est est -H ou -CH₃,
R₈ et R₉ sont indépendamment un groupe C₇ à C₂₀ alkyl substitué ou non, un groupe aryl substitué ou non, un groupe -aryl- (C₁ à C₂₀ alkyl) substitué ou non ou un groupe - (C₁ à C₂₀ alkyl)-aryl substitué ou non,
avec R₈ et/ou R₉ est différent de H.

De préférence, le motif de répétition comprenant un groupement hydrophobe est présent dans le copolymère entre 0,01 et 5 mol %, encore plus préférentiellement entre 0,1 et 1,5 mol %.

Dans un autre mode de réalisation particulier pouvant être combiné avec les précédents, le polymère ne comprend pas d'ammonium quaternaire.

Dans un autre mode de réalisation particulier pouvant être combiné avec les précédents, le polymère n'est pas un copolymère d'acide acrylamidométhylpropane sulfonique (AMPS), d'acide maléique et d'acide acrylique.

Dans un autre mode de réalisation particulier pouvant être combiné avec les précédents, le polymère n'est pas l'acide polyphosphinocarboxylique sulfonaté.

Selon l'invention le polymère a une masse moléculaire comprise entre 1 MDa et 30 MDa.

### Détection des additifs dans l'échantillon à analyser

Pour la détection des additifs utilisés pour la récupération assistée du pétrole et du gaz de schiste dans l'échantillon à analyser avec la méthode selon l'invention, on mesure la variation de fluorescence entre ce mélange comprenant des ions lanthanides éventuellement complexés avec un additif et un mélange contrôle (par exemple ne contenant pas d'additif ou contenant une quantité connue d'additif), par fluorescence en temps résolu.

Du fait de la diminution de l'effet quenching de l'eau sur le lanthanide en présence de l'additif, la variation de fluorescence par rapport à une solution de référence sans additif (ou une quantité connue d'additif) est ainsi directement et spécifiquement reliée à la présence des additifs utilisés pour la récupération assistée du pétrole et du gaz de schiste dans le fluide à analyser.

La comparaison des caractéristiques d'émission, d'excitation et/ou de durée de vie des ions lanthanides libres et des ions lanthanides complexés permet ainsi de détecter et, le cas échéant de quantifier les additifs utilisés pour la récupération assistée du pétrole et du gaz de schiste présents dans le fluide extrait.

Selon l'invention, l'additif utilisé pour la récupération assistée du pétrole et du gaz de schiste est détecté, et son taux quantifié, en utilisant une méthode de fluorescence en temps résolu qui est notamment décrite dans l'article "Ultrasensitive bioanalytical assays using time resolved fluorescence détection", Pharmacol. Ther. Vol. 66(2), pp. 207-35, 1995. Celle-ci repose sur l'application d'un délai, dit délai d'intégration, entre l'excitation de l'échantillon à analyser et la mesure du signal émis, de manière à s'affranchir des fluorescences parasites à durée de vie courte. Cette méthode peut être mise en oeuvre à température ambiante, notamment à l'aide d'un appareil de type Cary Eclipse de la société Agilent.

La longueur d'onde d'excitation peut être comprise entre 200 et 600 nm et la longueur d'onde d'émission peut être comprise entre 300 et 800 nm. Le délai d'intégration peut être compris entre 0,001 et 10 ms, de préférence entre 0,01 et 5 ms, plus préférentiellement entre 0,1 et 3 ms. Dans certains cas, plus ce délai est long, meilleur est le rapport signal / bruit, ce qui améliore la fiabilité de la mesure. La durée de récolte des photons peut aller de 0,1 à 10 ms, par exemple.

Cette méthode peut être appliquée de différentes manières. Il est ainsi possible de comparer l'intensité d'émission de l'échantillon testé avec celles obtenues à différentes concentrations d'additifs utilisés pour la récupération assistée du pétrole et du gaz de schiste, pour en déduire la concentration en additif de l'échantillon. En variante, il est possible de détecter plusieurs additifs utilisés pour la récupération assistée du pétrole et du gaz de schiste dans l'échantillon testé en mesurant la vitesse de décroissance du signal émis par l'échantillon, ou demi-vie, et en comparant les valeurs obtenues avec celles connues pour les différents additifs à détecter.

### FIGURES

La figure 1 représente la courbe de calibration du polymère 3630 avec Eu-2,5-diaminopyridine selon l'exemple 8.
La figure 2 représente la courbe de calibration du polymère AN977 avec Eu-2,5-diaminopyridine selon l'exemple 9.
La figure 3 représente la courbe de calibration du polymère AN125 avec Eu-2,5-diaminopyridine selon l'exemple 10.

La présente invention sera mieux comprise à la lumière des exemples non limitatifs suivants, qui sont donnés à titre purement illustratif et n'ont pas pour but de limiter la portée de cette invention qui est définie par les revendications annexées.

### EXEMPLES

### I - Préparation des solutions révélatrices et des solutions de polymères

### Exemple 1. Préparation d'une solution d'europium concentrée

200 mg de chlorure d'europium hexahydraté (EuCl₃.6H₂O, CAS n° 13759-92-7) sont pesés dans un flacon de 100 mL et 100 mL d'eau ultra-pure sont ajoutés. Une solution de chlorure d'europium hexahydraté à 2 000 ppm est obtenue.

### Exemple 2. Préparation d'une solution de 2,5-diaminopyridine concentrée

100 mg de 2,5-diaminopyridine dihydrochloride (C₅H₇N₃ • 2HCl, CAS n° 26878-35-3) sont pesés dans un flacon de 100 mL et 100 mL d'eau ultra-pure sont ajoutés. Une solution de 2,5-diaminopyridine dihydrochloride à 1 000 ppm est obtenue.

### Exemple 3. Préparation d'une solution d'HEPES concentrée (tampon)

1,191 g d'HEPES (C₈H₁₈N₂O₄S, CAS n° 7365-45-9) sont pesés dans un flacon de 100 mL et 100 mL d'eau ultra-pure sont ajoutés. Une solution d'HEPES à 11 910 ppm est obtenue.

### Exemple 4. Préparation d'une solution révélatrice

5 g de chlorure de sodium sont pesés dans un flacon de 250 mL et 219,4 mL d'eau ultra-pure sont ajoutés. Dans l'ordre, sont ensuite ajoutés : 25 mL de la solution d'HEPES préparée selon l'exemple 3, 625 µL de la solution d'europium préparée selon de l'exemple 1 et 5 mL de la solution de 2,5-diaminopyridine préparée selon l'exemple 2.

### Exemple 5. Préparation d'une solution mère de polymère anionique hydrosoluble 3630

Le polymère anionique hydrosoluble 3630 est un copolymère statistique d'acrylamide et d'acrylate de sodium (70/30 en mol%). Il s'agit d'un polymère linéaire ayant une masse molaire d'environ 18 MDa.

Une solution concentrée à 10 g/L de polymère anionique hydrosoluble 3630 dans de l'eau salée est préparée en dissolvant 1 g de polyacrylamide 3630 dans 100 mL d'eau salée (référencée à 6 g/L) . 1 mL de cette solution est prélevé et introduit dans un flacon de 100 mL et 99 mL d'eau salée (référencée à 6 g/L) sont ajoutés. Une solution à 100 ppm de polymère anionique hydrosoluble 3630 dans l'eau salée est obtenue.

### Exemple 6. Préparation d'une solution mère de polymère anionique hydrosoluble AN977

Le polymère anionique hydrosoluble AN977 est un copolymère statistique d'acrylamide et d'acrylate de sodium (34/66 en mol%). Il s'agit d'un polymère linéaire ayant une masse molaire d'environ 8 MDa.

Une solution à 100 ppm de polymère anionique hydrosoluble AN977 dans l'eau salée est préparée selon le mode opératoire décrit dans l'exemple 5 en utilisant 1 g de polyacrylamide AN977 à la place de 1 g de polyacrylamide 3630.

### Exemple 7. Préparation d'une solution mère de polymère anionique hydrosoluble AN125

Le polymère anionique hydrosoluble AN125 est un copolymère statistique d'acrylamide et d'acide acrylamido-2-méthyl-2-propane sulfonique (75/25 en mol%). Il s'agit d'un polymère linéaire ayant une masse molaire d'environ 8 MDa.

Une solution à 100 ppm de polymère anionique hydrosoluble AN125 dans l'eau salée est préparée selon le mode opératoire décrit dans l'exemple 5 en utilisant 1 g de polyacrylamide AN125 à la place de 1 g de polyacrylamide 3630.

### II - Détection et quantification des polymères

### Quantification des additifs par fluorescence en temps résolution (FTR) :

Les mesures sont effectuées sur un spectrofluorimètre Agilent Cary Eclipse. Le temps de vie de luminescence des terres rares augmente avec la diminution du nombre de molécules d'eau dans leur sphère de coordination. La complexation des terres rares par les polymères permet ainsi leur détection et leur quantification.

Les temps de vie de fluorescence de ces complexes sont typiquement de l'ordre de la milliseconde. Cette propriété permet notamment de les distinguer de la fluorescence des composés organiques qui est de l'ordre de la microseconde.

Les complexes d'europium possèdent quatre pics d'émission notables dans le visible : 536, 595, 614 et 650 nm. Les limites de l'appareillage (perte de sensibilité pour λem > 650 nm) nous ont conduites à quantifier ces entités via l'émission à 614 nm. L'intensité du pic est relative à la concentration, au degré de complexation et aux conditions de détection.

### Exemple 8. Quantification du polymère 3630 - Tracé d'une droite de calibration

Une gamme de solutions étalons 0 - 100 ppm est préparée par dilution avec de l'eau salée à 6 g/L de la solution à 100 ppm préparée lors de l'exemple 5. Chaque étalon est ensuite dilué par 10 dans la solution révélatrice préparée selon l'exemple 4. Pour cela, 9 mL de la solution révélatrice préparée selon l'exemple 4 sont prélevés et introduits dans un flacon de 10 mL. 1 mL de la solution étalon à doser est ajouté. Après 1h, 2,5 mL du mélange étalon - solution révélatrice sont prélevés puis introduits dans une cuve pour spectrophotomètre (ref : cuvette Sarstedt^{®} en PMMA 2,5-4,5 mL). Le contenu de la cuvette est finalement analysé en fluorescence en temps résolu.

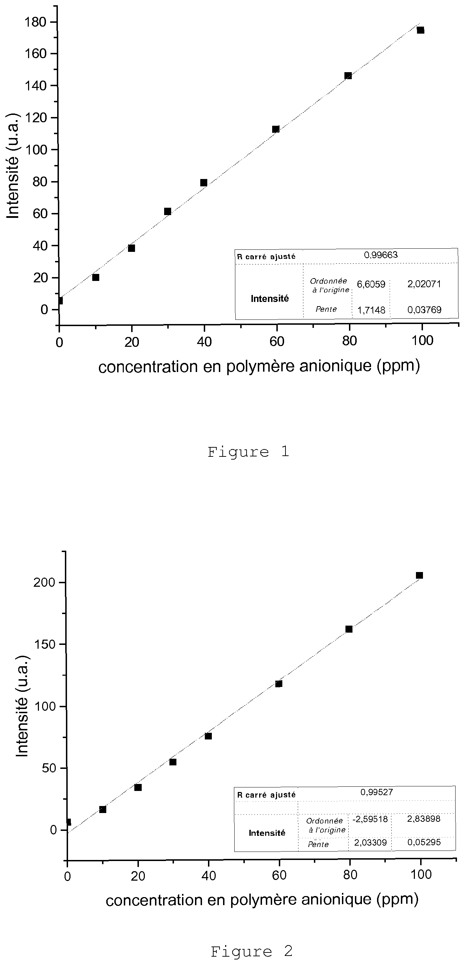

La figure 1 représente la droite de calibration obtenue. Ces données montrent qu'il est possible d'effectuer des analyses quantitatives d'additifs utilisés pour la récupération assistée du pétrole et du gaz de schiste dans un fluide aqueux.

### Exemple 9. Quantification du polymère AN977 - Tracé d'une droite de calibration

Une gamme de solutions étalons 0 - 100 ppm est préparée par dilution dans de l'eau salée à 6 g/L de la solution à 100 ppm préparée lors de l'exemple 6. Chaque étalon est ensuite dilué par 20 dans la solution révélatrice préparée selon l'exemple 4. Pour cela, 9,5 mL de la solution révélatrice de l'exemple 4 sont prélevés et introduits dans un flacon de 10 mL. 0,5 mL de la solution étalon à doser sont ajoutés. Après 1h, 2,5 mL du mélange étalon - solution révélatrice sont prélevés puis introduits dans une cuve pour spectrophotomètre (ref : cuvette Sarstedt^{®} en PMMA 2,5-4,5 mL). Le contenu de la cuvette est finalement analysé en fluorescence en temps résolu.

La figure 2 représente la droite de calibration obtenue. Ces données montrent qu'il est possible d'effectuer des analyses quantitatives d'additifs utilisés pour la récupération assistée du pétrole et du gaz de schiste dans un fluide aqueux.

### Exemple 10. Quantification du polymère AN125 - Tracé d'une droite de calibration

Une gamme de solutions étalons 0 - 60 ppm est préparée par dilution avec de l'eau salée à 6 g/L de la solution à 100 ppm préparée lors de l'exemple 7. Chaque étalon est ensuite dilué par 5 dans la solution révélatrice préparée selon l'exemple 4. Pour cela, 8 mL de la solution révélatrice de l'exemple 4 sont prélevés et introduits dans un flacon de 10 mL. 2 mL de la solution étalon à doser sont ajoutés. Après 1h, 2,5 mL du mélange étalon - solution révélatrice sont prélevés puis introduits dans une cuve pour spectrophotomètre (ref : cuvette Sarstedt^{®} en PMMA 2,5-4,5 mL). Le contenu de la cuvette est finalement analysé en fluorescence en temps résolu.

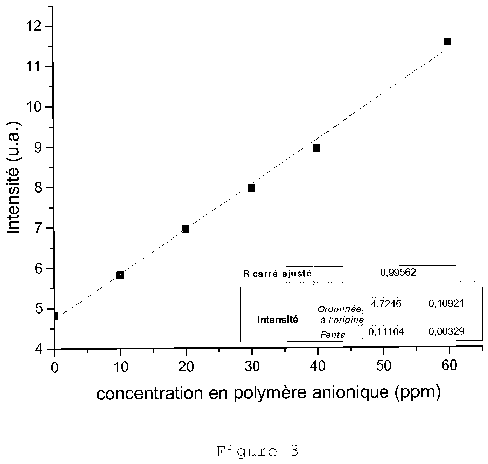

La figure 3 représente la droite de calibration obtenue. Ces données montrent qu'il est possible d'effectuer des analyses quantitatives d'additifs utilisés pour la récupération assistée du pétrole et du gaz de schiste dans un fluide aqueux.

## Revendications

1. Méthode de détection d'additifs utilisés pour la récupération assistée du pétrole et du gaz de schiste, dans des eaux d'injection ou des eaux de production, ladite méthode comprenant:
a. le mélange d'une solution révélatrice comprenant au moins un cation lanthanide et éventuellement un agent chélateur des lanthanides, avec un échantillon d'eaux d'injection ou d'eaux de production à analyser comprenant au moins un additif utilisé pour la récupération assistée du pétrole et du gaz de schiste, dans des conditions permettant la complexation du lanthanide par l'additif présent,
b. La détection et, le cas échéant, la quantification, de la variation de fluorescence liée à la présence de l'additif dans les eaux d'injection ou les eaux de production par fluorescence en temps résolu,
ledit additif utilisé pour la récupération assistée du pétrole et du gaz de schiste étant un polymère hydrosoluble ayant une masse molaire comprise entre 1 MDa et 30 MDa choisi parmi :
- les polymères comprenant au moins un motif de répétition comprenant une liaison amide ;
- les biopolymères anioniques tels que le xanthane ;
- les polymères cationiques, tel que le poly DADMAC.

2. Méthode selon la revendication 1 **caractérisée en ce que** ledit polymère comprenant au moins un motif de répétition comprenant une liaison amide comprend un motif de répétition de formule I 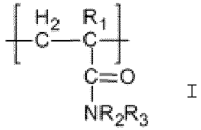
où R₁ est -H ou -CH₃,
R₂ est -H ou un groupe C₁ à C₄ alkyl substitué ou non,
R₃ est -H ou un groupe C₁ à C₄ alkyl substitué ou non, ou un groupe -L-R₄,
où L est une liaison ou un groupe C₁ à C₁₀ alkyl substitué ou non, interrompu par 0, 1 ou plusieurs liaisons -NR₂- ou -O- ou -S-, ou un groupe - (C₁ à C₁₀ alkyl substitué ou non) - (N⁺R₆R₇) - (C₁ à C₁₀ alkyl substitué ou non) - avec R₆ et R₇ qui sont indifféremment -H ou un groupe C₁ à C₄ alkyl substitué ou non,
et R₄ est -H ou un groupe carboxylate (-COO⁻) ou un groupe sulfonate (-SO₃⁻), avec éventuellement un contre-ion.

3. Méthode selon la revendication 2 **caractérisée en ce que** ledit polymère comprend en outre un motif de répétition de formule II 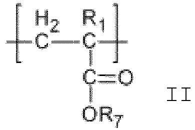
où R₁ est -H ou -CH₃,
et OR₇ est O-H ou O⁻ et un contre-ion.

4. Méthode selon la revendication 2 ou 3 **caractérisée en ce que** ledit polymère comprend en outre un motif de répétition de formule III 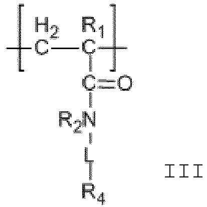
où R₁ est -H ou -CH₃,
R₂ est -H ou un groupe C₁ à C₄ alkyl substitué ou non,
L est une liaison ou un groupe C₁ à C₁₀ alkyl substitué ou non, interrompu par 0, 1 ou plusieurs liaisons -NR₂- ou - O- ou -S-, ou un groupe - (C₁ à C₁₀ alkyl substitué ou non) - (N⁺R₆R₇) - (C₁ à C₁₀ alkyl substitué ou non) - avec R₆ et R₇ qui sont indifféremment -H ou un groupe C₁ à C₄ alkyl substitué ou non,
et R₄ est -H ou un groupe carboxylate (-COO⁻) ou un groupe sulfonate (-SO₃⁻), avec éventuellement un contre-ion.

5. Méthode selon l'une des revendications 2 à 4 **caractérisée en ce que** ledit polymère comprend en outre un motif de répétition issu de la polymérisation d'un monomère non-ionique, de préférence ledit monomère non-ionique est choisi parmi acryloyl morpholine, N-vinylcaprolactame, N-vinylpyrrolidone, N,N-diméthylacrylamide, N-ispropylacrylamide, diacétone acrylamide, N-vinylformamide, N-vinyl acetamide, N-vinylpyridine, hydroxybutyl vinyl ether et isoprenol.

6. Méthode selon l'une des revendications 2 à 5 **caractérisée en ce que** ledit polymère comprend en outre un motif de répétition comprenant un groupement hydrophobe, de préférence de formule IV 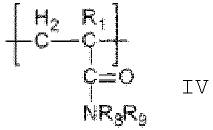
où R₁ est est -H ou -CH₃,
R₈ et R₉ sont indépendamment un groupe C₇ à C₂₀ alkyle substitué ou non, un groupe aryl substitué ou non, un groupe -aryl- (C₁ à C₂₀ alkyl) substitué ou non ou groupe - (C₁ à C₂₀ alkyl)-aryl substitué ou non,
avec R₈ et/ou R₉ est différent de H.

7. Méthode selon l'une des revendications 1 à 6, **caractérisée en ce que** la solution révélatrice comprend en outre au moins 1g/L d'ions chlorure, et de préférence une concentration en ions chlorure comprise entre 5 et 50g/L.

8. Méthode selon l'une des revendications 1 à 7, **caractérisée en ce que** la solution révélatrice comprend en outre au moins 1g/L d'un composé chimique utilisé dans la fabrication de solution tampon, par exemple l'acide 4-(2-hydroxyéthyl)-1-pipérazine éthane sulfonique (HEPES)ou l'acétate de sodium.

9. Méthode selon l'une des revendications 1 à 8, **caractérisée en ce que** le lanthanide est choisi parmi : Pr, Nd, Sm, Eu, Tb, Dy, Ho, Er, Tm et Yb, ainsi que leurs mélanges, de préférence Eu.

10. Méthode selon l'une des revendications 1 à 9 **caractérisée en ce que** ledit additif est présent à une concentration inférieure ou égale à 10 ppm dans l'échantillon d'eaux d'injection ou d'eaux de production à analyser, de préférence inférieure ou égale à 1 ppm.

## Patentansprüche

1. Verfahren zum Nachweis von Additiven, die für die unterstützte Gewinnung von Öl und Schiefergas verwendet werden, in Injektionswasser oder Produktionswasser, wobei das Verfahren Folgendes umfasst:
a. Mischen einer Entwicklerlösung, die mindestens ein Lanthanid-Kation und gegebenenfalls einen Chelatbildner für Lanthanide enthält, mit einer Probe des zu analysierenden Injektionswassers oder Produktionswassers, das mindestens ein Additiv enthält, das zur unterstützten Gewinnung von Öl und Schiefergas verwendet wird, unter Bedingungen, die eine Komplexierung des Lanthanids durch das vorhandene Additiv ermöglichen,
b. Nachweis und falls erforderlich Quantifizierung der Fluoreszenzänderung, die mit der Anwesenheit des Additivs im Injektionswasser oder Produktionswasser verbunden ist, durch zeitaufgelöste Fluoreszenz,
wobei das Additiv, das für die unterstützte Gewinnung von Öl und Schiefergas verwendet wird, ein wasserlösliches Polymer mit einer Molmasse zwischen einschließlich 1 MDa und 30 MDa ist, das ausgewählt ist aus:
- Polymeren, die mindestens eine Wiederholungseinheit mit einer Amidbindung enthalten;
- anionischen Biopolymeren wie Xanthan;
- kationischen Polymeren wie Poly DADMAC.

2. Verfahren nach Anspruch 1, **dadurch gekennzeichnet, dass** das Polymer, das mindestens eine Wiederholungseinheit mit einer Amidbindung umfasst, eine Wiederholungseinheit der Formel I umfasst 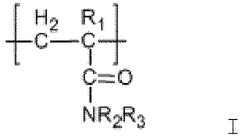 worin
R₁ -H oder -CH₃ ist,
R₂ -H oder eine substituierte oder unsubstituierte C₁-bis C₄-Alkylgruppe ist,
R₃ -H oder eine substituierte oder unsubstituierte C₁-bis C₄-Alkylgruppe oder eine Gruppe -L-R₄ ist, worin L eine Bindung oder eine substituierte oder unsubstituierte C₁- bis C₁₀-Alkylgruppe ist, die durch 0, 1 oder mehrere -NR₂- oder -O- oder -S- Bindungen unterbrochen ist, oder eine Gruppe -(substituiertes oder unsubstituiertes C₁- bis C₁₀-Alkyl)-(N⁺R₆R₇)- (substituiertes oder unsubstituiertes C₁- bis C₁₀-Alkyl)-, wobei R₆ und R₇ unterschiedslos -H oder eine substituierte oder unsubstituierte C₁- bis C₄-Alkylgruppe sind, und
R₄ -H oder eine Carboxylatgruppe (-COO⁻) oder eine Sulfonatgruppe (-SO₃⁻) ist, gegebenenfalls mit einem Gegenion.

3. Verfahren nach Anspruch 2, **dadurch gekennzeichnet, dass** das Polymer weiterhin eine Wiederholungseinheit der Formel II umfasst 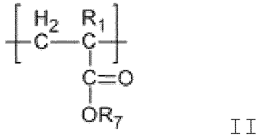 worin
R₁ -H oder -CH₃ ist, und
OR₇ O-H oder O⁻ und ein Gegenion ist.

4. Verfahren nach Anspruch 2 oder 3, **dadurch gekennzeichnet, dass** das Polymer ferner eine Wiederholungseinheit der Formel III umfasst 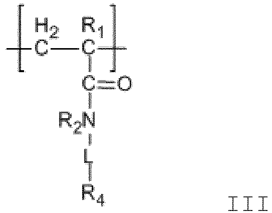 worin
R₁ -H oder -CH₃ ist,
R₂ -H oder eine substituierte oder unsubstituierte C₁-bis C₄-Alkylgruppe ist,
L eine Bindung oder eine substituierte oder unsubstituierte C₁- bis C₁₀-Alkylgruppe ist, die durch 0, 1 oder mehrere -NR₂- oder -O- oder -S- Bindungen unterbrochen ist, oder eine Gruppe -(substituiertes oder unsubstituiertes C₁- bis C₁₀-Alkyl)-(N⁺R₆R₇)- (substituiertes oder unsubstituiertes C₁- bis C₁₀-Alkyl)-, wobei R₆ und R₇ unterschiedslos -H oder eine substituierte oder unsubstituierte C₁- bis C₄-Alkylgruppe sind, und
R₄ -H oder eine Carboxylatgruppe (-COO⁻) oder eine Sulfonatgruppe (-SO₃⁻) ist, gegebenenfalls mit einem Gegenion.

5. Verfahren nach einem der Ansprüche 2 bis 4, **dadurch gekennzeichnet, dass** das Polymer zusätzlich eine Wiederholungseinheit umfasst, die aus der Polymerisation eines nichtionischen Monomers stammt, vorzugsweise ist das nichtionische Monomer ausgewählt aus Acryloylmorpholin, N-Vinylcaprolactam, N- Vinylpyrrolidon, N, N-Dimethylacrylamid, N-Isopropylacrylamid, Diacetonacrylamid, N-Vinylformamid, N-Vinylacetamid, N-Vinylpyridin, Hydroxybutylvinylether und Isoprenol.

6. Verfahren nach einem der Ansprüche 2 bis 5, **dadurch gekennzeichnet, dass** das Polymer weiterhin eine Wiederholungseinheit umfasst, die eine hydrophobe Gruppe vorzugsweise der Formel IV umfasst 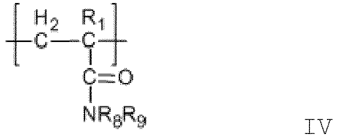 worin
R₁ -H oder -CH₃ ist,
R₈ und R₉ unabhängig voneinander eine substituierte oder unsubstituierte C₇- bis C₂₀-Alkylgruppe, eine substituierte oder unsubstituierte Arylgruppe, eine substituierte oder unsubstituierte -Aryl-(C₁- bis C₂₀-Alkyl)-Gruppe oder eine substituierte oder unsubstituierte - (C₁- bis C₂₀-Alkyl) -Arylgruppe sind, wobei R₈ und/oder R₉ von H verschieden ist.

7. Verfahren nach einem der Ansprüche 1 bis 6, **dadurch gekennzeichnet, dass** die Entwicklerlösung weiterhin mindestens 1 g/L Chloridionen umfasst und vorzugsweise eine Chloridionenkonzentration zwischen einschließlich 5 und 50 g/L aufweist.

8. Verfahren nach einem der Ansprüche 1 bis 7, **dadurch gekennzeichnet, dass** die Entwicklerlösung weiterhin mindestens 1 g/L einer chemischen Verbindung umfasst, die bei der Herstellung von Pufferlösungen verwendet wird, z.B. 4-(2-Hydroxyethyl)-1-piperazinethan-sulfonsäure (HEPES) oder Natriumacetat.

9. Verfahren nach einem der Ansprüche 1 bis 8, **dadurch gekennzeichnet, dass** das Lanthanid ausgewählt ist aus: Pr, Nd, Sm, Eu, Tb, Dy, Ho, Er, Tm und Yb sowie deren Mischungen, vorzugsweise Eu.

10. Verfahren nach einem der Ansprüche 1 bis 9, **dadurch gekennzeichnet, dass** das Additiv in der zu analysierenden Probe des Injektionswassers oder Produktionswassers in einer Konzentration von weniger als oder gleich 10 ppm vorliegt, vorzugsweise weniger als oder gleich 1 ppm.

## Claims

1. Method for detecting additives used in the enhanced recovery of oil and shale gas, in injection water or production water, said method comprising:
a. mixing a detecting solution comprising at least one lanthanide cation and optionally a chelating agent of the lanthanides, with a sample of injection water or of production water to be analysed comprising at least one additive used in the enhanced recovery of oil and shale gas, under conditions allowing complexing of the lanthanide by the additive present,
b. detecting and, if appropriate, quantifying the variation in fluorescence associated with the presence of the additive in the injection water or the production water by time-resolved fluorescence,
said additive used in the enhanced recovery of oil and shale gas being a water-soluble polymer having a molecular weight comprised between 1 MDa and 30 MDa selected from:
- polymers comprising at least one repeat unit comprising an amide bond;
- anionic biopolymers such as xanthan;
- cationic polymers, such as poly DADMAC.

2. Method according to claim 1, **characterized in that** said polymer comprising at least one repeat unit comprising an amide bond comprises a repeat unit of formula I 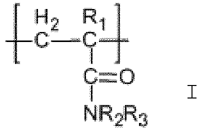
where R₁ is -H or -CH₃,
R₂ is -H or a substituted or unsubstituted C₁ to C₄ alkyl group,
R₃ is -H or a substituted or unsubstituted C₁ to C₄ alkyl group, or an-L-R₄ group,
where L is a bond or a substituted or unsubstituted C₁ to C₁₀ alkyl group, interrupted by 0, 1 or more -NR₂- or -O- or -S- bonds, or a -(substituted or unsubstituted C₁ to C₁₀ alkyl)-(N⁺R₆R₇)- (substituted or unsubstituted C₁ to C₁₀ alkyl)- group with R₆ and R₇ which are either -H or a substituted or unsubstituted C₁ to C₄ alkyl group,
and R₄ is -H or a carboxylate group (-COO⁻) or a sulphonate group (-SO₃⁻), optionally with a counter-ion.

3. Method according to claim 2, **characterized in that** said polymer moreover comprises a repeat unit of formula II 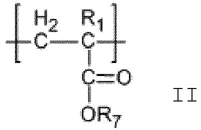
where R₁ is -H or -CH₃,
and OR₇ is O-H or O⁻ and a counter-ion.

4. Method according to claim 2 or 3, **characterized in that** said polymer moreover comprises a repeat unit of formula III 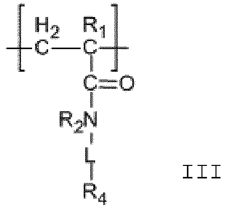
where R₁ is -H or -CH₃,
R₂ is -H or a substituted or unsubstituted C₁ to C₄ alkyl group,
L is a bond or a substituted or unsubstituted C₁ to C₁₀ alkyl group, interrupted by 0, 1 or more -NR₂- or -O- or - S- bonds, or a -(substituted or unsubstituted C₁ to C₁₀ alkyl)-(N⁺R₆R₇) - (substituted or unsubstituted C₁ to C₁₀ alkyl)- group with R₆ and R₇ which are either -H or a substituted or unsubstituted C₁ to C₄ alkyl group,
and R₄ is -H or a carboxylate group (-COO⁻) or a sulphonate group (-SO₃⁻), optionally with a counter-ion.

5. Method according to one of claims 2 to 4, **characterized in that** said polymer moreover comprises a repeat unit originating from the polymerization of a non-ionic monomer, preferably said non-ionic monomer is selected from acryloyl morpholine, N-vinylcaprolactam, N-vinylpyrrolidone, N,N-dimethylacrylamide, N-ispropylacrylamide, diacetone acrylamide, N-vinylformamide, N-vinylacetamide, N-vinylpyridine, hydroxybutyl vinyl ether and isoprenol.

6. Method according to one of claims 2 to 5, **characterized in that** said polymer moreover comprises a repeat unit comprising a hydrophobic group, preferably of formula IV 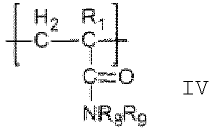
where R₁ is -H or -CH₃,
R₈ and R₉ are independently a substituted or unsubstituted C₇ to C₂₀ alkyl group, a substituted or unsubstituted aryl group, a substituted or unsubstituted -aryl-(C₁ to C₂₀ alkyl) group or substituted or unsubstituted - (C₁ to C₂₀ alkyl)-aryl group,
where R₈ and/or R₉ are different from H.

7. Method according to one of claims 1 to 6, **characterized in that** the detecting solution moreover comprises at least 1g/L of chloride ions, and preferably a concentration of chloride ions comprised between 5 and 50g/L.

8. Method according to one of claims 1 to 7, **characterized in that** the detecting solution moreover comprises at least 1g/L of a chemical compound used in the production of buffer solution, for example 4-(2-hydroxyethyl)-1-piperazine-ethanesulphonic acid (HEPES) or sodium acetate.

9. Method according to one of claims 1 to 9, **characterized in that** the lanthanide is selected from: Pr, Nd, Sm, Eu, Tb, Dy, Ho, Er, Tm and Yb, as well as mixtures thereof, preferably Eu.

10. Method according to one of claims 1 to 9, **characterized in that** said additive is present at a concentration less than or equal to 10 ppm in the sample of injection water or of production water to be analysed, preferably less than or equal to 1 ppm.
